# EUROPEAN PATENT APPLICATION

(11) **EP 4 650 040 A1**
(43) Date of publication of application: **19.11.2025**
(21) Application number: 24741552.4
(22) Date of filing: 11.01.2024
(51) Int. Cl.: B01D 71/52, A61M 1/18, A61M 1/36, B01D 63/00, B01D 69/00, B01D 69/02, B01D 71/44, B01D 71/68, B01D 71/80, B01D 71/82

(54) **SEMIPERMEABLE MEMBRANE AND SEMIPERMEABLE MEMBRANE MODULE**

(30) Priority: 13.01.2023 JP 2023003986
(71) Applicant: Toyobo Co., Ltd., Kita-ku Osaka-shi, Osaka 530-0001 (JP)
(72) Inventor: KANAI, Taisei, Otsu-shi, Shiga 520-0292 (JP); KOYAMA, Shinya, Otsu-shi, Shiga 520-0292 (JP)
(74) Representative: Vossius & Partner Patentanwälte Rechtsanwälte mbB
(86) International application number: PCT/JP2024/000353
(87) International publication number: WO 2024/150780

(57) **Abstract**

A semipermeable membrane comprising a sulfonated polyarylene ether copolymer, wherein the sulfonated polyarylene ether copolymer includes a hydrophobic segment repeating unit represented by a formula (1) and a hydrophilic segment repeating unit represented by a formula (2) as copolymerization components, and a solvation energy density of the entirety of the semipermeable membrane is from 170 to 225 kJ·mol⁻¹·nm⁻³.

## Description

### TECHNICAL FIELD

The present invention relates to a semipermeable membrane and a semipermeable membrane module.

### BACKGROUND ART

Blood purification therapy based on membrane separation techniques such as hemodialysis, hemofiltration, and hemodiafiltration has been increasingly employed in recent years not only for patients with chronic renal failure but also for patients with acute renal failure, and also widely employed in critical care, ICU, and the like.

By the way, infection with COVID-19 can be accompanied by acute renal disorders with a sudden loss of kidney function and/or by a cytokine storm which is a severe immune reaction against viruses in which an excessive amount of proinflammatory mediators circulate in blood. Among patients with serious symptoms of COVID-19, 15 to 30% develop acute renal disorders and 67% develop organ dysfunction syndrome which is considered ascribable to an excessive amount of cytokines in circulation.

There is a therapy known as continuous renal replacement therapy (CRRT), which is for acute renal failure, organ dysfunction syndrome, and the like and which involves performing body fluid correction over a prolonged period of time in a slow manner (at a low circulation flow rate) as compared to the therapy conditions for ordinary hemodialysis and hemofiltration. CRRT is employed not only for replacing renal functions such as fluid removal, waste removal, and electrolyte balance correction but also for removing proinflammatory mediators, and, for instance, CRRT can be carried out as a single-round treatment at a blood flow rate from 50 to 150 mL/minute and a filtration rate of 5 to 50 mL/minute over 24 to 48 hours.

As a semipermeable membrane (a blood treatment apparatus) for CRRT, SepXiris (registered trademark) manufactured by Baxter Limited is known, for example. The semipermeable membrane used in SepXiris contains, as a component, a copolymer of acrylonitrile and sodium methallyl sulfonate. It is conceivable that this semipermeable membrane is mainly capable of adsorbing positively charged cytokines because its negatively charged sulfonate groups can form ionic bonds with amino groups of positively charged cytokines (see The Journal of Artificial Organs, vol. 43, No. 3, pp. 233-237, 2014 (NPL 1)).

Moreover, Japanese Patent No. 6817240 (Japanese Patent Laying-Open No. 2018-171431 (PTL 1)) discloses a hollow fiber membrane for blood purification that has hemocompatibility and cytokine adsorption capabilities. This hollow fiber membrane contains polymethyl methacrylate and sodium p-styrenesulfonate, and due to the negatively charged sulfonate groups, it is conceivable that the membrane is mainly capable of adsorbing positively charged cytokines.

Moreover, Japanese Patent Laying-Open No. 2001-70767 (PTL 2) discloses an ultrafiltration membrane for surface water treatment comprising a composition made of polyethersulfone, polyvinylpyrrolidone, and sulfonated polyethersulfone, which is, however, an ultrafiltration membrane for the purpose of water treatment and not accompanied by any mention regarding medical membranes.

Moreover, International Patent Laying-Open No. WO 2018/025979 (PTL 3) recites that a copolymer having a hydration energy density and the like within certain ranges is excellent in inhibiting adhesion of proteins and the like, and it also recites that a copolymer of this type is usable as a material of medical separation membranes.

### CITATION LIST

### PATENT LITERATURE

PTL 1: Japanese Patent No. 6817240 (Japanese Patent Laying-Open No. 2018-171431)
PTL 2: Japanese Patent Laying-Open No. 2001-70767
PTL 3: International Patent Laying-Open No. WO 2018/025979

### NON PATENT LITERATURE

NPL 1: The Journal of Artificial Organs, vol. 43, No. 3, pp. 233-237, 2014

### SUMMARY OF INVENTION

### TECHNICAL PROBLEM

The above-mentioned conventional semipermeable membrane for continuous renal replacement therapy is highly capable of adsorbing (highly capable of removing) positively charged substances, but it is not very capable of adsorbing negatively charged substances, which is a drawback.

Hence, an object of the present invention is to provide a semipermeable membrane that is highly capable of adsorbing proinflammatory mediators (both negatively charged substances and positively charged substances).

### SOLUTION TO PROBLEM

[1] A semipermeable membrane comprising:
   a sulfonated polyarylene ether copolymer, wherein
   the sulfonated polyarylene ether copolymer includes a hydrophobic segment repeating unit represented by a formula (1) below and a hydrophilic segment repeating unit represented by a formula (2) below as copolymerization components; 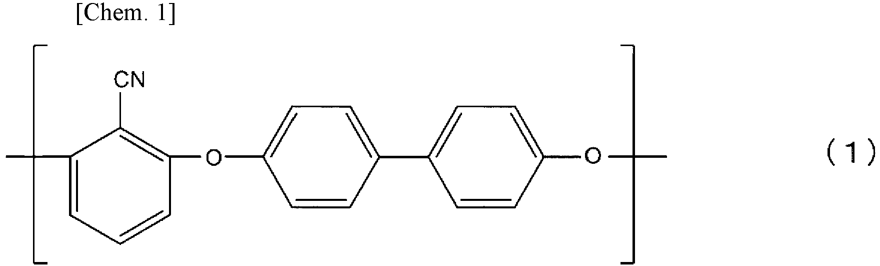 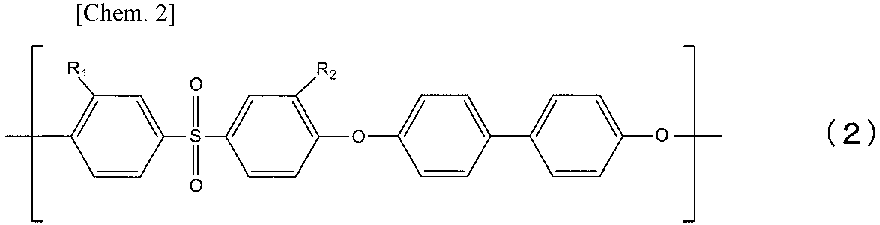
   where each of R₁ and R₂ represents "-SO₃M" and M represents a metallic element, and
   a solvation energy density of the entirety of the semipermeable membrane is from 170 to 225 kJ·mol⁻¹·nm⁻³.
[2] The semipermeable membrane according to [1], wherein a component ratio (in mole) of the formula (1) is from 0.40 to 0.70, a component ratio (in mole) of the formula (2) is from 0.30 to 0.60, and a sum of the component ratio (in mole) of the formula (1) and the component ratio (in mole) of the formula (2) is 1.00.
[3] The semipermeable membrane according to [1] or [2], comprising polyethersulfone, the sulfonated polyarylene ether copolymer, and polyvinylpyrrolidone.
[4] The semipermeable membrane according to [3], wherein a content ratio of (polyethersulfone)/(sulfonated polyarylene ether copolymer)/(polyvinylpyrrolidone) in the entirety of the semipermeable membrane (in mass) is (75 to 90)/(7 to 22)/(3 to 18).
[5] The semipermeable membrane according to any one of [1] to [4], for continuous renal replacement therapy.
[6] The semipermeable membrane according to any one of [1] to [5], having a structure that is not uniform in a thickness direction.
[7] A semipermeable membrane module comprising the semipermeable membrane according to any one of [1] to [6].
[8] The semipermeable membrane module according to [7], having a structure that is not uniform in a thickness direction.

### ADVANTAGEOUS EFFECTS OF INVENTION

The present invention makes it possible to provide a semipermeable membrane that is highly capable of adsorbing both negatively charged substances and positively charged substances.

### BRIEF DESCRIPTION OF DRAWINGS

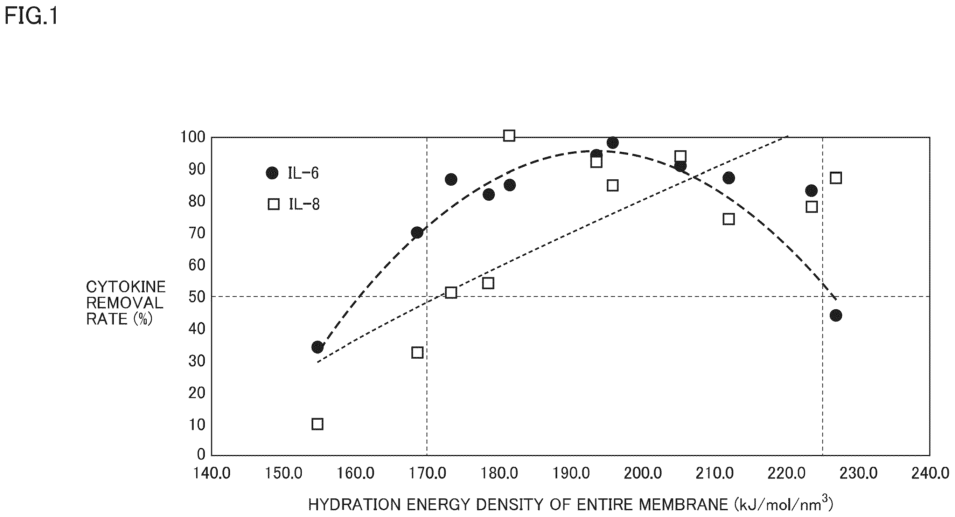

Fig. 1 is a graph showing the relationship between the solvation energy density of the entire membrane and the cytokine removal rate in Examples.

### DESCRIPTION OF EMBODIMENTS

In the following, a description will be given of embodiments of the present invention, but the scope of the present invention is not limited to these embodiments.

### <Semipermeable Membrane>

### <<Solvation Energy Density>>

The solvation energy density of the entire semipermeable membrane according to the present embodiment is from 170 to 225 kJ·mol⁻¹·nm⁻³, preferably from 175 to 220 kJ·mol⁻¹·nm⁻³, more preferably from 178 to 215 kJ·mol⁻¹·nm⁻³, further preferably from 180 to 210 kJ·mol⁻¹·nm⁻³.

Herein, solvation energy means the amount of change in the energy that the system can obtain at the time when a target substance (solute) is added to water (solvent). As the unit of solvation energy, J·mol⁻¹ is used, for example.

The solvation energy density of a copolymer is calculated by a mathematical expression (1) below.

### [Math. 1]

In the mathematical expression (1), "Solvation energy of monomer unit i" refers to the absolute value of a value obtained by subtracting the energy of the monomer unit i in vacuum from the energy of the monomer unit i in water; N represents the total number of monomer species constituting the copolymer; and i represents an integer from 1 to N.

The solvation energy density of a homopolymer is also calculated by the mathematical expression (1) where N is 1 (in other words, a mathematical expression (2) below). Solvation energy density of homopolymer (kJ · mol-1 · nm-3) = { Solvation energy of monomer unit}/{Volume of monomer unit}

"Solvation energy of monomer unit" refers to the absolute value of a value obtained by subtracting the energy of the monomer unit in vacuum from the energy of the monomer unit in water.

The energy of the monomer unit in vacuum and the energy of the monomer unit in water can be calculated by a method below.

Firstly, the molecule model of the monomer unit is subjected to geometry optimization. In the geometry optimization, the density functional theory (DFT) is used. B3LYP is used as the functional, and 6-31G(d, p) is used as the basis function. As the keyword for the input file, opt is set.

Then, for the structure after geometry optimization, the energy in vacuum and the energy in water are calculated.

For calculation of the energy in vacuum, the density functional theory is used. B3LYP is used as the functional, and 6+31G(d, p) is used as the basis function.

For calculation of the energy in water, the density functional theory is used. B3LYP is used as the functional, and 6+31G(d, p) is used as the basis function. Furthermore, for calculation of the energy in water, a continuum dielectric model is used, where the keywords are as follows.
SCRF=(PCM, G03Defaults, Read, Solvent=Water)
Radii=UAHF
Alpha=1.20

"Solvation energy of monomer unit" is determined as the absolute value of a value obtained by subtracting the energy of the monomer unit in vacuum (SCF energy) from the energy of the monomer unit in water (SCF energy). SCF energy is the value of E which appears on the row with "SCF Done:".

The volume of the monomer unit can be calculated by, for example, COSMO calculation with the use of a quantum chemistry calculation software "Gaussian16" manufactured by Gaussian. For geometry optimization in COSMO calculation, the density functional theory is used. BVP86 is used as the functional, TZVP is used as the basis function, and DGA1 is used as the fitting basis. Furthermore, the keywords are as follows.
SCRF=(CPCM, READ)
Radii=klamt

The structure after optimization in COSMO calculation can be subjected to a single point calculation to calculate the volume. BVP86 is used as the functional, TZVP is used as the basis function, and DGA1 is used as the fitting basis. Furthermore, the keyword is as follows.

### SCRF=COSMORS

"Molar fraction of monomer unit" in the mathematical expression (1) can be calculated from the peak area obtained by measurement on a nuclear magnetic resonance (NMR) apparatus. In the case when the above-mentioned molar fraction cannot be calculated by NMR measurement due to, for example, overlaps between peaks, the above-mentioned molar fraction may be calculated by elemental analysis.

### (Solvation Energy Density of Entire Semipermeable Membrane)

Herein, the solvation energy density of the entirety of the semipermeable membrane, namely, the solvation energy density of the entire semipermeable membrane is the volume average solvation energy density of the components constituting the semipermeable membrane.

For instance, when the semipermeable membrane is composed of polyethersulfone, a sulfonated polyarylene ether copolymer (SPN), and polyvinylpyrrolidone, the solvation energy density of the entire semipermeable membrane is the volume average value of the solvation energy density of SPN calculated by the mathematical expression (1), the solvation energy density of the polyethersulfone calculated by the mathematical expression (2), and the solvation energy density of the polyvinylpyrrolidone calculated by the mathematical expression (2).

Generally, solvation energy tends to be great when more polar functional groups such as carbonyl groups (such as ester groups and amido groups, for example) are present in the molecule than alkyl groups. When there is no difference in solvation energy, the smaller the volume of the monomers is, the greater the numerical value of the solvation energy density is.

Hence, the solvation energy density of the entire semipermeable membrane can be regulated by changing the types of the polymers constituting the semipermeable membrane and the molar fractions of such polymers.

For instance, when the semipermeable membrane is composed of polyethersulfone, SPN, and polyvinylpyrrolidone, by changing the molar fractions of the polyethersulfone, the SPN, and the polyvinylpyrrolidone, it is possible to regulate the solvation energy density of the entire semipermeable membrane.

The solvation energy density of the entirety of a copolymer (such as SPN, for example) can be regulated by changing the molar fractions of the monomer units constituting the copolymer.

### <<Composition of Semipermeable Membrane>>

The semipermeable membrane according to the present embodiment includes a sulfonated polyarylene ether copolymer (which may be abbreviated as "SPN" hereinafter). SPN is a negatively charged polymer.

Preferably, the semipermeable membrane according to the present embodiment includes polyethersulfone (a hydrophobic polymer), a sulfonated polyarylene ether copolymer (SPN: a negatively charged polymer), and polyvinylpyrrolidone (a hydrophilic polymer).

### (Polyethersulfone)

The polyethersulfone is a compound that includes a structural unit represented by the following formula (3). The polyethersulfone is expected to act to adsorb proinflammatory mediators via hydrophobic interaction.

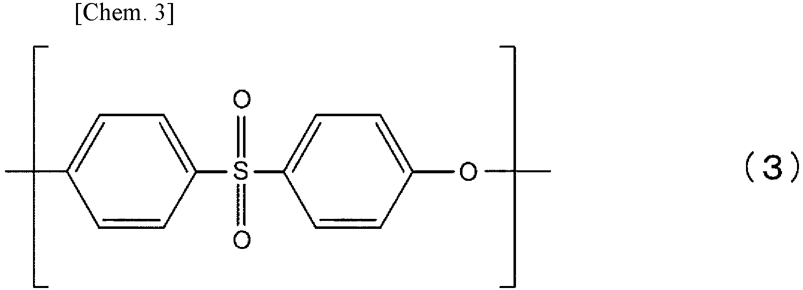

### (Sulfonated Polyarylene Ether Copolymer)

The sulfonated polyarylene ether copolymer is a compound that includes a hydrophobic segment repeating unit represented by the following formula (1) and a hydrophilic segment repeating unit represented by the following formula (2).

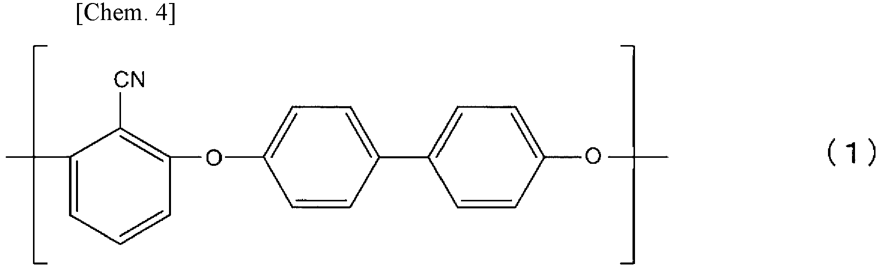

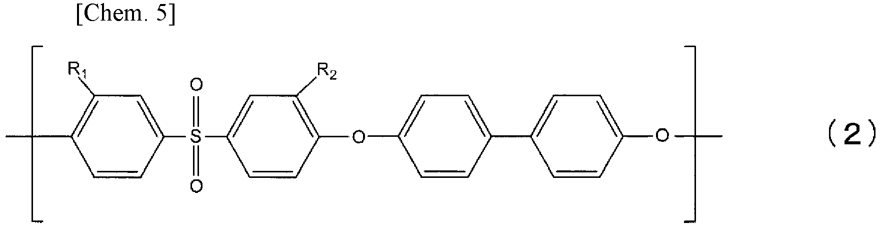

In the formula (2), R₁ and R₂ independently represent "-SO₃M" or "-SO₃H". Here, M represents a metallic element.

The component ratio (in mole) of the formula (1) is preferably from 0.40 to 0.70, more preferably from 0.45 to 0.67, further preferably from 0.50 to 0.60.

The component ratio (in mole) of the formula (2) is preferably from 0.30 to 0.60, more preferably from 0.33 to 0.55, further preferably from 0.40 to 0.50.

The sum of the component ratio (in mole) of the formula (1) and the component ratio (in mole) of the formula (2) is 1.00.

It is conceivable that when the component ratios fall within the above-mentioned ranges, due to the effects of both the hydrophilic segment and the hydrophobic segment, the SPN present on the surface of the semipermeable membrane (and the water adsorbed thereto) can interact with positively charged substances and negatively charged substances (and the water adsorbed thereto) to a proper degree and, as a result, a semipermeable membrane that is highly capable of adsorbing both positively charged substances and negatively charged substances may be obtained.

The sulfonated polyarylene ether copolymer may be any of a random copolymer, a block copolymer, an alternating copolymer, and the like.

M (a metallic element) is not particularly limited, and examples thereof include sodium, potassium, and lithium.

In the sulfonated polyarylene ether copolymer, it is more preferable that each of R₁ and R₂ in the formula (2) be "-SO₃Na" or "-SO₃K".

The sulfonated polyarylene ether copolymer is a material that does not change much during a long-term use of the membrane.

The sulfonated polyarylene ether copolymer may be obtained by a conventionally known method and the like. For instance, 2,6-dichlorobenzonitrile, 3,3'-disulfo-4,4'-dichlorodiphenyl sulfone metal salt, and 4,4'-biphenol may be subjected to reaction in the presence of a basic compound for polymerization via an aromatic nucleophilic substitution reaction, and thereby a copolymer consisting of a hydrophobic segment represented by the formula (1) and a hydrophilic segment represented by the formula (2) may be obtained.

The polymerization may be caused at a temperature within the range of 0 to 350°C, and, preferably, the temperature is from 50 to 250°C. At below 0°C, the reaction tends not to proceed sufficiently, and at above 350°C, polymer degradation tends to begin.

The reaction may be caused in the absence of solvent, but preferably caused in solvent. Examples of the solvent that can be used include N-methyl-2-pyrrolidone, N,N-dimethylacetamide, N,N-dimethylformamide, dimethyl sulfoxide, diphenyl sulfone, and sulfolane; however, these examples are not restrictive and any solvent that is stable in an aromatic nucleophilic substitution reaction may be used.

Examples of the basic compound include sodium hydroxide, potassium hydroxide, sodium carbonate, potassium carbonate, sodium hydrogen carbonate, potassium hydrogen carbonate, and the like; however, these examples are not restrictive and those that can transform an aromatic diol into an active phenoxide structure may be used.

In an aromatic nucleophilic substitution reaction, water can be produced as a byproduct. In that case, toluene and/or the like may be made to coexist in the reaction system in addition to and independently from the polymerization solvent to remove water as an azeotrope, out of the system. As the method for removing water out of the system, use of a water-absorbing material such as molecular sieves may also be adopted.

In the case when the aromatic nucleophilic substitution reaction is caused in a solvent, it is preferable that the monomers be added in such a manner that a resulting polymer concentration from 5 to 50 mass% is to be achieved. When it is below 5 mass%, the degree of polymerization tends not to increase. When it is above 50 mass%, the viscosity of the reaction system tends to become too high to perform posttreatment of the reaction product with ease.

After the completion of the polymerization reaction, the solvent is removed from the reaction solution by evaporation, followed by rinsing the remaining product as needed, to obtain a desired polymer (a sulfonated polyarylene ether copolymer). Alternatively, the reaction solution may be added to a solvent that does not dissolve the polymer very well, to make the polymer precipitated as solid, followed by collection of the precipitate by filtration, and thereby the polymer may be obtained.

### (Polyvinylpyrrolidone)

The polyvinylpyrrolidone is a polymer of monomers including at least N-vinylpyrrolidone.

The polyvinylpyrrolidone preferably includes a structural unit represented by the following formula (4).

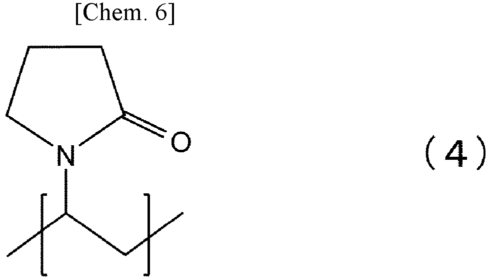

As the polyvinylpyrrolidone, products from BASF with weight average molecular weights of 9,000 (K17), 450,000 (K60), and/or 1,200,000 (K90) can be used, for example.

Preferably, the content ratio of (polyethersulfone)/(sulfonated polyarylene ether copolymer)/(polyvinylpyrrolidone) in the entirety of the semipermeable membrane is (75 to 90)/(7 to 22)/(3 to 18) (in mass%).

Preferably, the semipermeable membrane has a structure that is not uniform in a thickness direction (an asymmetric structure). More preferably, it has a structure that has a dense layer on the inner surface side thereof, with the pore size increasing continuously or discontinuously from the inner surface toward the outer surface (an asymmetric structure).

Examples of the semipermeable membrane having an asymmetric structure (an asymmetric membrane) include a membrane in which the above-mentioned dense layer serves as a separation-activating layer that substantially determines the pore size of the semipermeable membrane. It is impossible to directly measure the pore size of the dense layer, and the molecular weight cut-off of SC dextran measured with the use of a semipermeable membrane having a dense layer is defined as the pore size of the dense layer. In the present invention, the molecular weight cut-off of SC dextran measured in this manner is preferably 100 kDa or less. From the viewpoint of durability of the membrane (filtration stability), the semipermeable membrane is preferably an asymmetric membrane.

It should be noted that the components of the semipermeable membrane having an asymmetric structure may vary in the thickness direction of the membrane.

In a hollow fiber membrane having an asymmetric structure, the aperture ratio of the inner surface is preferably different from the aperture ratio of the outer surface. The aperture ratio of the outer surface of the hollow fiber membrane is preferably 29% or more, more preferably 30% or more. The aperture ratio of the outer surface of the hollow fiber membrane is determined in an image that is taken with a scanning electron microscope (SEM) described below.

Examples of the semipermeable membrane include semipermeable membranes called nanofiltration membranes (NF membranes), ultrafiltration membranes (UF membranes), and microfiltration membranes (MF membranes). The semipermeable membrane is preferably an ultrafiltration membrane.

Usually, the pore size of an NF membrane is from about 1 to 2 nm, the pore size of an UF membrane is from about 2 to 100 nm, and the pore size of an MF membrane is about 0.1 µm or more.

The shape of the semipermeable membrane is not particularly limited. The semipermeable membrane may be a flat membrane or a hollow fiber membrane (a hollow-fiber-type semipermeable membrane), and is preferably a hollow fiber membrane. A hollow fiber membrane provides an increased membrane area per module as compared to a flat membrane, which is an advantage.

Preferably, the semipermeable membrane according to the present embodiment is a semipermeable membrane for continuous renal replacement therapy, capable of removing proinflammatory mediators.

Proinflammatory mediators are biologically active substances (pain-producing substances, prostanoids, cytokines) that are released by leucocytes, mast cells, macrophages and the like infiltrated in damaged tissue and inflammation sites. Examples of proinflammatory mediators include positively charged substances and negatively charged substances.

Examples of the positively charged substances include interleukin-8 (IL-8), interleukin-10 (IL-10), transforming growth factor β (TGF-β), monocyte chemotaxis promoting factor (MCP1), platelet-derived growth factor (PDGF), and the like.

Examples of the negatively charged substances include interleukin-6 (IL-6), interleukin-18 (IL-18), interleukin-1β (IL-1β), HMGB1 (High Mobility Group Box 1), tumor necrosis factor-α (TNF-α), and the like.

### <Semipermeable Membrane Module>

The present invention also relates to a semipermeable membrane module (a membrane separation instrument, a membrane separation apparatus) comprising the above-described semipermeable membrane. The semipermeable membrane module is not particularly limited as long as it is a separation instrument (a separation apparatus) capable of performing separation (such as solid-liquid separation and liquid-liquid separation) with the use of the above-mentioned semipermeable membrane.

In the semipermeable membrane module (such as a continuous hemofiltration instrument described above, for example) having the above-mentioned hollow fiber membrane, it is preferable that the dialysate be made to flow on the outer side of the hollow fiber membrane and blood be made to flow on the inner side (inside the hollow portion) of the hollow fiber membrane.

### [Examples]

In the following, a more detailed description will be given of the present invention by way of Examples, but these Examples are not intended to limit the scope of the present invention.

### (Preparation of Sulfonated Polyarylene Ether Copolymer (SPN))

3,3'-disulfo-4,4'-dichlorodiphenyl sulfone disodium salt, 2,6-dichlorobenzonitrile, 4,4'-biphenol, potassium carbonate, and molecular sieves were weighed into a four-necked flask, through which nitrogen was passed. NMP was added thereto and the resultant was stirred at 150°C for 50 minutes, followed by increasing the reaction temperature to the range of 195°C to 200°C to continue reaction until the viscosity of the system rose sufficiently. The resultant was left to cool, and after cooling, the sedimented molecular sieves were removed and the polymer was precipitated in water. The polymer thus obtained was rinsed in boiling water for 1 hour, followed by careful rinsing in pure water to completely remove the remaining potassium carbonate. Subsequently, the polymer from which potassium carbonate had been removed was dried, and thereby a sulfonated polyarylene ether copolymer (SPN) was obtained.

SPN (a copolymer of the formula (1) and the formula (2)) used in Examples and Comparative Examples is one of SPN15, SPN33, SPN44, SPN55, and SPN65 specified in Table 1. These SPNs were prepared by changing the ratio between 3,3'-disulfo-4,4'-dichlorodiphenyl sulfone disodium salt and 2,6-dichlorobenzonitrile used in the preparation of SPN.

As for SPN15, the component molar ratio of (1) is 0.85 and the component molar ratio of (2) is 0.15.

As for SPN33, the component molar ratio of (1) is 0.67 and the component molar ratio of (2) is 0.33.

As for SPN44, the component molar ratio of (1) is 0.56 and the component molar ratio of (2) is 0.44.

As for SPN55, the component molar ratio of (1) is 0.45 and the component molar ratio of (2) is 0.55.

As for SPN65, the component molar ratio of (1) is 0.35 and the component molar ratio of (2) is 0.65.

### (Preparation of Hollow Fiber Membranes of Examples 1 to 3 and Comparative Examples 4 and 5)

Polyethersulfone (PES, manufactured by Sumitomo Chemical), sulfonated polyarylene ether copolymer (SPN obtained in the above-described manner), polyvinylpyrrolidone K-90 (PVP, manufactured by Nippon Shokubai), 46.2 mass% of N-methyl-2-pyrrolidone (NMP, manufactured by Mitsubishi Chemical), and 30.8 mass% of triethylene glycol (TEG, manufactured by Mitsubishi Chemical) were heated and dissolved into uniformity. The ratio between the polyethersulfone (PES), the sulfonated polyarylene ether copolymer (SPN), and the polyvinylpyrrolidone (PVP) are as specified in Table 1.

The spinning dope thus obtained, together with a hollow-forming material (NMP/TEG/water=12/8/80), was discharged through a tube-in-orifice nozzle that was warmed to 55°C, passed through a 200-mm dry section that was shut off from the outside air by a spinning tube, coagulated in a coagulation bath (NMP/TEG/water=24/16/60) at 74°C, rinsed in a water bath at 80°C, and wound on a skeiner at a spinning rate of 37 m/min. The hollow fiber membranes (a bundle) thus obtained were rinsed with warm water at 70°C for removal of excess solvent and the like, and then immersed in 60-mass% glycerol solution at 60°C so that the pores were filled with the aqueous glycerol solution. Centrifugation for liquid removal was performed to remove excess aqueous glycerol solution adhered to the surface of the hollow fiber membranes, followed by drying at 60°C.

The hollow fiber membrane thus obtained had a dense layer on the inner surface side thereof, an inner diameter of 0.240 mm, and an outer diameter of 0.350 mm.

### (Preparation of Hollow Fiber Membranes of Examples 4 to 6)

Polyethersulfone (PES, manufactured by Sumitomo Chemical), sulfonated polyarylene ether copolymer (SPN obtained in the above-described manner), polyvinylpyrrolidone K-90 (PVP, manufactured by Nippon Shokubai), 46.8 mass% of N-methyl-2-pyrrolidone (NMP, manufactured by Mitsubishi Chemical), and 31.2 mass% of triethylene glycol (TEG, manufactured by Mitsubishi Chemical) were heated and dissolved into uniformity. The ratio between the polyethersulfone (PES), the sulfonated polyarylene ether copolymer (SPN), and the polyvinylpyrrolidone (PVP) are as specified in Table 1. Except these, the same conditions as in Example 1 were employed to produce hollow fiber membranes.

### (Preparation of Hollow Fiber Membranes of Examples 7 to 12 and Comparative Examples 1, 2, 6, 7)

Polyethersulfone (PES, manufactured by Sumitomo Chemical), sulfonated polyarylene ether copolymer (SPN obtained in the above-described manner), polyvinylpyrrolidone K-90 (PVP, manufactured by Nippon Shokubai), 48.0 mass% of N-methyl-2-pyrrolidone (NMP, manufactured by Mitsubishi Chemical), and 32.0 mass% of triethylene glycol (TEG, manufactured by Mitsubishi Chemical) were heated and dissolved into uniformity. The ratio between the polyethersulfone (PES), the sulfonated polyarylene ether copolymer (SPN), and the polyvinylpyrrolidone (PVP) are as specified in Table 1. Except these, the same conditions as in Example 1 were employed to produce hollow fiber membranes.

### (Preparation of Hollow Fiber Membrane of Example 13)

Polyethersulfone (PES, manufactured by Sumitomo Chemical), sulfonated polyarylene ether copolymer (SPN obtained in the above-described manner), polyvinylpyrrolidone K-90 (PVP, manufactured by Nippon Shokubai), 47.4 mass% of N-methyl-2-pyrrolidone (NMP, manufactured by Mitsubishi Chemical), and 31.6 mass% of triethylene glycol (TEG, manufactured by Mitsubishi Chemical) were heated and dissolved into uniformity. The ratio between the polyethersulfone (PES), the sulfonated polyarylene ether copolymer (SPN), and the polyvinylpyrrolidone (PVP) are as specified in Table 1. Except these, the same conditions as in Example 1 were employed to produce a hollow fiber membrane.

### (Preparation of Hollow Fiber Membrane of Example 14)

Polyethersulfone (PES, manufactured by Sumitomo Chemical), sulfonated polyarylene ether copolymer (SPN obtained in the above-described manner), polyvinylpyrrolidone K-90 (PVP, manufactured by Nippon Shokubai), 46.8 mass% of N-methyl-2-pyrrolidone (NMP, manufactured by Mitsubishi Chemical), and 31.2 mass% of triethylene glycol (TEG, manufactured by Mitsubishi Chemical) were heated and dissolved into uniformity. The ratio between the polyethersulfone (PES), the sulfonated polyarylene ether copolymer (SPN), and the polyvinylpyrrolidone (PVP) are as specified in Table 1. Except these, the same conditions as in Example 1 were employed to produce a hollow fiber membrane.

### (Preparation of Hollow Fiber Membrane of Comparative Example 3)

Polyethersulfone (PES, manufactured by Sumitomo Chemical), sulfonated polyarylene ether copolymer (SPN obtained in the above-described manner), polyvinylpyrrolidone K-90 (PVP, manufactured by Nippon Shokubai), 45.0 mass% of N-methyl-2-pyrrolidone (NMP, manufactured by Mitsubishi Chemical), and 30.0 mass% of triethylene glycol (TEG, manufactured by Mitsubishi Chemical) were heated and dissolved into uniformity. The ratio between the polyethersulfone (PES), the sulfonated polyarylene ether copolymer (SPN), and the polyvinylpyrrolidone (PVP) are as specified in Table 1. Except these, the same conditions as in Example 1 were employed to produce a hollow fiber membrane.

The composition (the composition ratio) of the components constituting each of the semipermeable membranes of Examples and Comparative Examples as well as the solvation energy density of the entirety of the semipermeable membranes (and the solvation energy density of SPN) are as specified in Table 1. The solvation energy density is measured by the above-described method.

### (Preparation of Semipermeable Membrane Module)

The hollow fiber membrane bundle obtained in the above-described manner was placed inside a tubular vessel equipped with two nozzles respectively for liquid introduction and liquid discharge, and from both ends, urethane resin was injected. Subsequently, the cured urethane resin parts were cut off so as to open the ends of the hollow fiber membranes at the end faces, and thereby a semipermeable membrane module was obtained which had a membrane area (the inner surface) of 1.7 m². Then, to each end of the semipermeable membrane module, a header cap equipped with a nozzle for blood introduction (or for blood discharge) was attached.

### <Evaluation Test>

### (Measurement of Cytokine Removal Rate)

For the semipermeable membrane module (a hollow fiber membrane module) prepared with the use of the hollow fiber membranes of Examples and Comparative Examples, the rate of removal of cytokines (IL-6 and IL-8) was measured. More specifically, the cytokine removal rate was measured in the manner described below.

To 1000 mL of porcine whole blood containing heparin added thereto, 24 µg of each of IL-6 and IL-8 was added to prepare a sample blood.

In the semipermeable membrane module (a continuous hemofiltration instrument) of Examples and Comparative Examples, the sample blood was circulated for 1 hour under conditions of a blood flow rate (Qb) of 100 mL/minute and a filtrate flow rate (Qf) of 10 mL/minute.

After circulation, the entire amount of the sample blood was collected, and the amounts of IL-6 and IL-8 in the sample blood were measured by enzyme-linked immunosorbent assay (ELISA). From the measured values and the amounts added to the sample blood, decrements of IL-6 and IL-8 were calculated, which were regarded as the removal rates (adsorption rates) of IL-6 and IL-8, respectively.

Results of measurement of the cytokine removal rate are shown in Table 1. Empty cells in Table 1 mean that no measurement was performed.

Fig. 1 is a graph showing the relationship between the solvation energy density of the entire membrane (semipermeable membrane) and the cytokine removal rate, only for those that have the cytokine removal rate measurement result entered in Table 1.

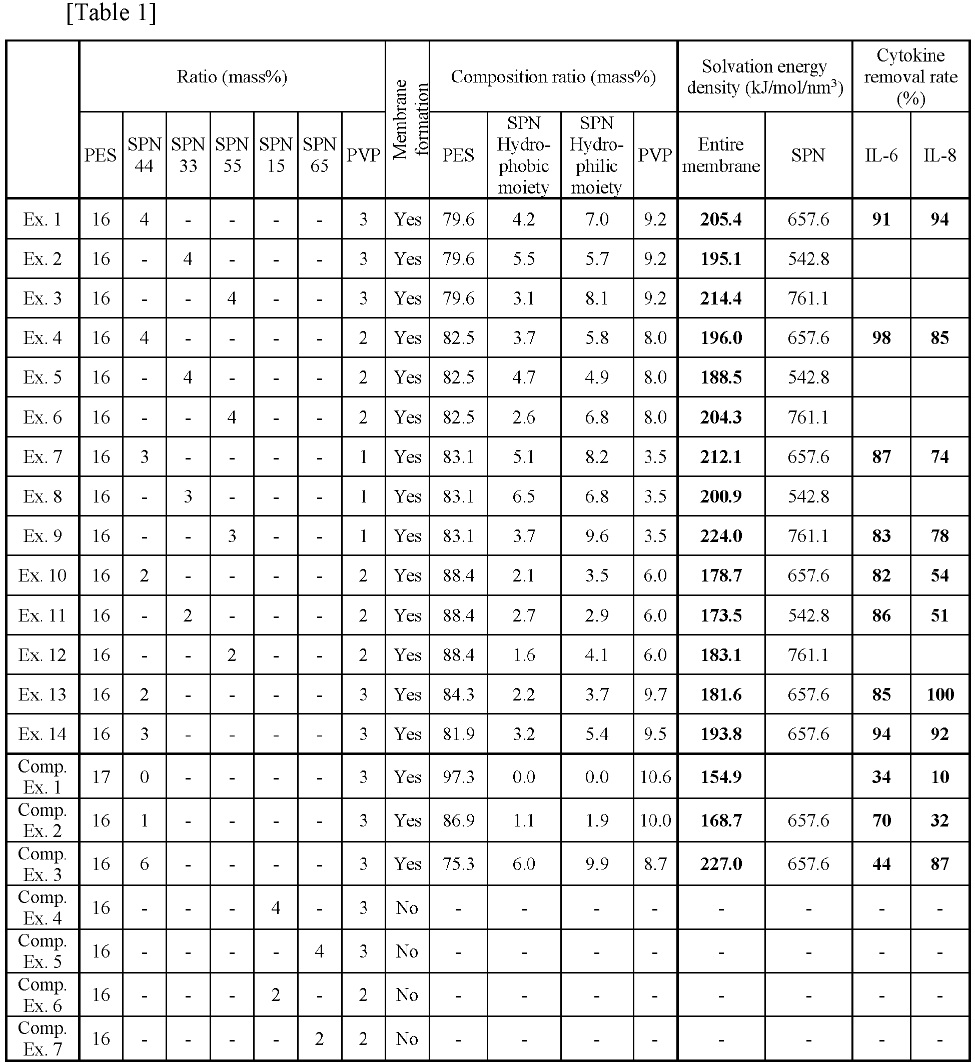

The results shown in Table 1 and Fig. 1 show that the IL-8 removal rate is 50% or more when the solvation energy density of the entire membrane (semipermeable membrane) is 170 kJ·mol⁻¹·nm⁻³ or more.

Probably, this occurs as a result of the following: since IL-8 is positively charged, when the ratio of the hydrophilic moiety of the sulfonated polyarylene ether copolymer (SPN) (the hydrophilic segment repeating unit represented by the formula (2)) increases (namely, when the solvation energy density of the entire semipermeable membrane increases), the amount of IL-8 adsorbed on the semipermeable membrane increases.

It is also shown that the IL-6 removal rate is 50% or more when the solvation energy density of the entire semipermeable membrane is from 170 to 225 kJ·mol⁻¹·nm⁻³.

Probably, this occurs as a result of the following: since IL-6 is negatively charged, due to the hydrophobic interaction between the semipermeable membrane and IL-6 and also due to the entrance of IL-6 into the pores of the semipermeable membrane, IL-6 is removed. Therefore, when the ratio of the hydrophobic moiety of SPN (the hydrophobic segment repeating unit represented by the formula (1) below) increases (namely, when the solvation energy density of the entire semipermeable membrane decreases), the amount of IL-6 adsorbed on the semipermeable membrane increases; however, when the ratio of the hydrophobic moiety of SPN becomes too high, the influence of electrostatic repulsion due to sulfonic groups becomes significant and thereby the amount of IL-6 adsorbed on the semipermeable membrane decreases.

In particular, it is shown that when the solvation energy density of the entire membrane is within the range of about 170 to 180 kJ·mol⁻¹·nm⁻³, due to the low solvation energy density, the IL-8 removal rate is significantly low.

Based on these findings, the present invention sets a lower limit to the range of the solvation energy density of the entire semipermeable membrane, and within this range, high adsorption capabilities (removal performance) are exhibited to both negatively charged substances (for example, negatively charged cytokines such as IL-6) and positively charged substances (for example, positively charged cytokines such as IL-8).

## Claims

1. A semipermeable membrane comprising:
a sulfonated polyarylene ether copolymer, wherein
the sulfonated polyarylene ether copolymer includes a hydrophobic segment repeating unit represented by a formula (1) below and a hydrophilic segment repeating unit represented by a formula (2) below as copolymerization components; 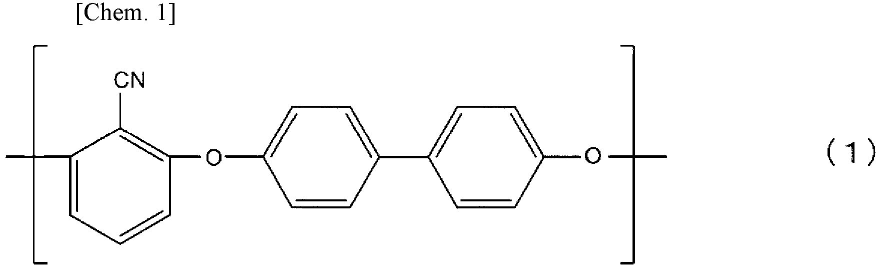 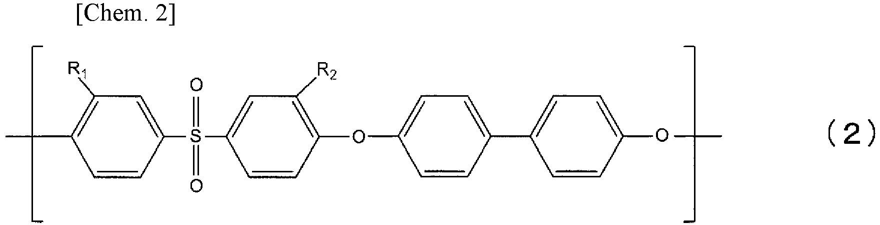
where each of R₁ and R₂ represents "-SO₃M" and M represents a metallic element, and
a solvation energy density of the entirety of the semipermeable membrane is from 170 to 225 kJ·mol⁻¹·nm⁻³.

2. The semipermeable membrane according to claim 1, wherein a component ratio (in mole) of the formula (1) is from 0.40 to 0.70, a component ratio (in mole) of the formula (2) is from 0.30 to 0.60, and a sum of the component ratio (in mole) of the formula (1) and the component ratio (in mole) of the formula (2) is 1.00.

3. The semipermeable membrane according to claim 1 or 2, comprising polyethersulfone, the sulfonated polyarylene ether copolymer, and polyvinylpyrrolidone.

4. The semipermeable membrane according to claim 3, wherein a content ratio of (polyethersulfone)/(sulfonated polyarylene ether copolymer)/(polyvinylpyrrolidone) in the entirety of the semipermeable membrane (in mass) is (75 to 90)/(7 to 22)/(3 to 18).

5. The semipermeable membrane according to any one of claims 1 to 4, for continuous renal replacement therapy.

6. The semipermeable membrane according to any one of claims 1 to 5, having a structure that is not uniform in a thickness direction.

7. A semipermeable membrane module comprising the semipermeable membrane according to any one of claims 1 to 6.

8. The semipermeable membrane module according to claim 7, for continuous renal replacement therapy.
